# EUROPÄISCHE PATENTSCHRIFT

(11) **EP 2 066 186 B1**
(45) Veröffentlichungstag und Bekanntmachung des Hinweises auf die Patenterteilung: **06.11.2013**
(21) Anmeldenummer: 07818394.4
(22) Anmeldetag: 25.09.2007
(51) Int. Cl.: A23L 1/30, A23F 3/18, A23F 3/38, A61K 36/82, A61P 3/10, A61P 25/28

(54) **GRÜNTEE-EXTRAKT, INSBESONDERE ZUR VERWENDUNG ALS FUNKTIONELLES LEBENSMITTEL, NAHRUNGSERGÄNZUNGSMITTEL ODER ENTSPRECHENDE ZUTAT, DESSEN VERWENDUNG SOWIE VERFAHREN ZUR HERSTELLUNG EINES SOLCHEN GRÜNTEE-EXTRAKTS**
GREEN TEA EXTRACT, ESPECIALLY FOR USE AS A FUNCTIONAL FOOD ITEM, FOOD SUPPLEMENT OR CORRESPONDING INGREDIENT, THE USE THEREOF AND METHOD FOR PRODUCING SAID GREEN TEA EXTRACT
EXTRAIT DE THÉ VERT, UTILISÉ EN PARTICULIER COMME ALIMENT FONCTIONNEL, COMPLÉMENT ALIMENTAIRE OU INGRÉDIENT CORRESPONDANT, SON UTILISATION ET PROCÉDÉ DE PRODUCTION ASSOCIÉ

(30) Priorität: 26.09.2006 DE 102006045763
(43) Veröffentlichungstag der Anmeldung: 10.06.2009
(73) Patentinhaber: Plantextrakt GmbH & Co. KG, 91487 Vestenbergsgreuth (DE)
(72) Erfinder: KLER, Adolf, 91413 Neustadt/Aisch (DE); ZENGER, Reinhold, 91325 Adelsdorf (DE); DIMPFEL, Wilfried, 35578 Wetzlar (DE)
(74) Vertreter: Hübner, Gerd
(86) Internationale Anmeldenummer: PCT/EP2007/008309
(87) Internationale Veröffentlichungsnummer: WO 2008/037417

(56) Entgegenhaltungen:
- EP-A- 0 552 519
- EP-A- 0 986 958
- EP-A- 1 393 726
- EP-A- 1 618 793
- WO-A-2006/033300
- DE-A1- 10 106 216
- JP-A- 2006 238 815
- KUHR S ET AL: "BESTIMMUNG VOM FLAVANOLEN, THEOGALLIN, GALLUSSAEURE UND COFFEIN IN TEE MITTELS HPLC DETERMINATION OF FLAVANOLS THEOGALLIN GALLIC ACID AND CAFFEINE IN TEA USING HLPC" ZEITSCHRIFT FUER LEBENSMITTELUNTERSUCHUNG UND -FORSCHUNG, XX, XX, Bd. 192, Nr. 6, 1991, Seiten 526-529, XP009094190 ISSN: 0044-3026

## Beschreibung

Die Erfindung betrifft einen Grüntee-Extrakt, insbesondere zur Verwendung als funktionelles Lebensmittel, Nahrungsergänzungsmittel oder entsprechende Zutat dazu, sowie dessen Verwendung und ein Verfahren zur Herstellung eines solchen Grüntee-Extrakts.

Zum Hintergrund der Erfindung ist festzuhalten, dass ein sich wandelndes Ernährungsverhalten, ein neues Gesundheitsbewusstsein, verknüpft mit dem Wunsch nicht nur wohlschmeckende, sichere Lebensmittel, sondern auch Lebensmittel mit bestimmten positiven ernährungsphysiologischen Eigenschaften zur Verfügung zu haben, feststellen lässt.

Neben bisher allgemein bekannten und beschriebenen Nährstoffen wie z.B. Eiweiß, Fett, Kohlenhydrate, Ballaststoffe, Vitamine, Mineralstoffe etc., spielen neuerdings auch sogenannte sekundäre Pflanzeninhaltsstoffe (SPS) eine zunehmend wichtigere Rolle. Dabei handelt es sich um eine große Anzahl verschiedenster chemischer Verbindungen, bzw. Verbindungsklassen wie z.B. Carotinoide, Terpene, Phytosterine, Polyphenole etc., wie sie auch von der Deutschen Gesellschaft für Ernährung (DGE) im Ernährungsbericht 1996 beschrieben worden sind.

Viele Pflanzen, insbesondere auch Kräuter bzw. Tee, sind reich an sekundären Pflanzeninhaltsstoffen und stellen deshalb ein geeignetes Ausgangsmaterial zur Herstellung von Pflanzenextrakten mit SPS dar. Diese Extrakte können als Lebensmittel oder Lebensmittelzutat zur Herstellung verschiedenster Lebensmittel, wie z.B. Getränke, Milchprodukte, Backwaren, Süßwaren etc., verwendet werden und geben dem verzehrfertigen Lebensmittel gewisse, funktionelle Eigenschaften.

Insbesondere Tee und Grüntee, beides Produkte des Teestrauches (Camellia sinensis / Camellia assamica bzw. deren Hybriden) sind reich an SPS, so z.B. an bekannten Stoffen wie Koffein, bzw. Polyphenolen, deren physiologische Wirkungen allgemein bekannt sind. Schwarztee und Grüntee sowie deren Extrakte enthalten darüberhinaus weitere ernährungsphysiologisch hochinteressante Verbindungen, wie z.B. Aminosäuren, Theanin, Theogallin.

Bei Theanin handelt es sich um ein Derivat der Glutaminsäure, dem 5-N-Ethyl-L-Glutamin. Für Theanin sind bestimmte ernährungsphysiologische Wirkungen bereits beschrieben. Im Wesentlichen sind dies relaxierende, stress-inhibierende, beruhigende Eigenschaften. Es existieren jedoch auch Hinweise auf anregende Eigenschaften.

Bei Theogallin handelt es sich um einen Ester der Gallussäure mit Chinasäure, der 5-Galloyl-Chinasäure. Über Theogallin und dessen ernährungsphysiologische Wirkungen im Teegetränk bzw. in Tee-Extrakten ist nichts bekannt.

Zum Stand der Technik lässt sich feststellen, dass die allgemein bekannten anregenden Eigenschaften von Koffein bereits seit langem beschrieben sind. Bei ständigem, insbesondere höherem Koffeingenuss existieren jedoch auch die für Koffein beschriebenen Nachteile wie Schlaflosigkeit, innere Unruhe, Verminderung des zerebralen Blutflusses etc. (siehe Hager-ROM 2004 Hagers Handbuch der Drogen und Arzneistoffe).

Für Theanin als Reinsubstanz werden einerseits beruhigende (siehe JP 2005 289 948 A1, JP 2005 232 045 A1 oder JP 9012454 A1), andererseits auch anregende Eigenschaften (siehe JP 9100230 A1) beschrieben. Dies gilt insbesondere für Theanin in Mischung mit Koffein, Arginin, Rice-Bran-extract (siehe JP 2002 3220 53 A1, KR 10 2005 121 535 A1 oder US 2005 0020 627 A1).

Aus der DE 101 06 216 A1 ist ein Grüntee- Extrakt bekannt, der entkoffeiniert, gerbstoffarm, jedoch bezüglich L-Theanin angereichert ist. Er wird hergestellt, indem Grünteeblätter, Grünteefannings oder Grünteepulver zunächst entkoffeiniert und anschließend in Wasser extrahiert werden. Der Extrakt wird daraufhin mit Polystyrol in Kontakt gebracht und anschließend aufkonzentriert. Der Extrakt wird verwendet als Zutat zu Getränken oder Getränkekonzentraten.

Aus der EP 10 57 483 A1 ist eine pharmazeutisch wirksame Zusammensetzung zur Behandlung verschiedener Symptome, wie Fettsucht, prämenstruale Symptome oder Kälteempfindlichkeit bekannt, die Theanin enthält.

Aus der EP 10 74 252 B1 ist eine Theanin enthaltende Zusammensetzung zur Unterdrückung von Verhaltensstörungen bei Haustieren bekannt. Die EP 0 986 958 A2 offenbart ein Verfahren zur Herstellung eines trinkfertigen entkoffeinierten Teeaufgusses, wobei eine feste Mischung aus trockenen, gegebenenfalls zerkleinerten, fermentierten oder teil- oder unfermentierten Teeblättern mit einem festen, teilchenförmigen, wasserunlöslichen Adsorptionsmittel für Koffein mit heiBem Wasser aufgegossen und 2-6 Minuten ziehengelassen wird, wonach die feste Mischung von dem so hergestellten Teeaufguß abgetrennt wird.

In der Patentliteratur werden ferner bereits verschiedenste Pflanzen und Pflanzen-Extrakte beschrieben, die positive Auswirkungen auf den Stoffwechsel eines Menschen haben, so z.B. in der koreanischen Patentanmeldung KR 1020030056987A eine Zusammensetzung mit Grüntee-Catechin als Wirkstoff für die Prophylaxe von Herz- und Gefäßerkrankungen, die mit Diabetes verbunden sind.

Dies ist von Bedeutung, da die Lebensumstände insbesondere in hochzivilisierten Staaten mit einseitiger Ernährung sowie Bewegungsmangel nach allgemeiner Auffassung als wesentliche Ursache für Übergewicht, Diabetes sowie weitergehende stoffwechselbedingte Erkrankungen zu sehen sind. Dies spiegelt sich in einer Zunahme von Diabetes-II-Erkrankungen in der Bevölkerung wieder.

Ziel, um dieser Tendenz entgegen zu wirken, muss eine ausgewogene Ernährung sowie ausreichende Bewegung sein. In diesem Umfeld können funktionelle Lebensmittel im Sinne einer Prophylaxe dazu beitragen oder zumindest dabei helfen, den Blutzuckerspiegel gefährdeter Personen in einem physiologischem Bereich zu halten.

Der vorliegenden Erfindung liegt nun die Aufgabe zugrunde, einen speziellen Grüntee-Extrakt zu entwickeln, der als breitbandig einsetzbares funktionales Lebensmittel beispielsweise eine Steigerung der cognitiven Leistungsfähigkeit bei gleichzeitiger Relaxation bewirkt und der als funktionelles Lebensmittel oder entsprechende Lebensmittelzutat in Getränken, Milchprodukten, Süßwaren, etc. eingesetzt werden kann. Damit wird das Ziel verfolgt, funktionelle Lebensmittel zu entwickeln, die insbesondere geistig arbeitenden Personen eine verbesserte und verlängerte cognitive Leistungsfähigkeit bei gleichzeitiger Relaxation garantieren soll. Eine weitere interessante Aufgabenstellung ist die mögliche positive Beeinflussung des menschlichen Glukose-Haushalts durch einen solchen Grüntee-Extrakt.

Diese Aufgabe wird durch einen Grüntee-Extrakt mit der in Patentanspruch 1 angegebenen Zusammensetzung in produkttechnischer Hinsicht gelöst. Versuche mit einem entkoffeinierten, Theanin- und Theogallin- angereicherten Grüntee-Extrakt bevorzugt mit einem Anteil von mindestens 6,0% Theanin, mindestens 3,0% Theogallin und maximal 0,03% Koffein haben überraschender Weise gezeigt, dass bei Applikation eines solchen Extraktes beispielsweise in einem Erfrischungsgetränk eine Steigerung der cognitiven Leistungsfähigkeit bei gleichzeitiger Relaxation festgestellt werden kann.

Überraschenderweise haben ferner Studien, wie z.B. ein Screening verschiedenster Pflanzen-Extrakte zum Thema "Blutzuckerregulierung, antidiabctische Eigenschaften von Pflanzenextrakten" ergeben, dass der erfindungsgemäße Gruntee-Extrakt auch Blutzucker-regulierende Eigenschaften aufweist. Es hat sich herausgestellt, dass dieser Theanin- und Theogallin-angereicherte Grüntee-Extrakt, entkoffeiniert, in der Lage ist, die Glukoseaufnahme durch differenzierte Adipocyten markant zu steigern.

Als bevorzugte Anreicherungsgrade für Theanin und Theogallin haben sich mindestens sechs Prozent bzw. mindestens drei Prozent erwiesen, wobei der Koffeingehalt bei maximal 0,03 Prozent liegen soll.

In verfahrenstechnischer Hinsicht gibt die Erfindung ein Verfahren zur Herstellung eines Grüntee-Extrakts an, das folgende Verfahrensschritte aufweist:
a) wässrige Extraktion von Grüntee-Blättern,
b) Flüssig-Flüssig-Extraktion des in Schritt a) erhaltenen wässrigen Grüntee-Extraktes mittels Ethylacetat unter Entfernung von Koffein und einem Teil der Polyphenole,
c) absorptive Anreicherung des in Schritt b) gewonnenen, wässrigen, mit Theanin und Theogallin angereicherten Extraktes in einem mit funktionalisiertem Divinylbenzol befüllten Absorber,
d) Elution des Absorbers mit demineralisiertem Wasser, wobei zwei separate Teilfraktionen erhalten werden, und
e) Aufkonzentrieren des Eluats der zweiten, an Theanin, Theogallin und Grüntee-spezifischen Aminosäuren reichen Teilfraktion zu einem entkoffeinierten, mit Theanin und Theogallin angereicherten Grüntee-Extrakt mit einem Trockensubstanz-Gehalt von vorzugsweise mind. 50 %.

Auf der Basis dieser grundsätzlichen Verfahrensschritte, die in den weiteren abhängigen Ansprüchen näher spezifiziert sind, können die oben erwähnten Anteile von Theanin, Theogallin und Koffein erreicht werden.

Weitere Merkmale, Einzelheiten und Vorteile der Erfindung werden aus der folgenden Beschreibung deutlich, die sich bezieht auf ein

### Ausführungsbeispiel:

Zur Herstellung des erfindungsgemäßen Extraktes wird Grüntee als Rohstoff verwendet. Dieser wird in einem ersten Schritt einer wässrigen Extraktion unterworfen - Schritt a) des Herstellungsverfahrens.

Der so erhaltene wässrige Grüntee-Extrakt wird in einem zweiten Schritt einer Flüssig-Flüssig-Extraktion - Schritt b) - mittels Ethylacetat unterworfen. Hierbei werden Koffein und ein Teil der Polyphenole entfernt, was zu einer Anreicherung an den gewünschten Stoffen Theanin und Theogallin führt.

Eine weitere, adsorptive Anreicherung erfolgt in einer dritten Prozessstufe - Schritt c) - über einen mit funktionalisiertem Divinylbenzol befüllten Adsorber. Der dabei anfallende, wässrige Durchlauf ist zu verwerfen. Die Elution - Schritt d) - erfolgt mit demineralisiertem Wasser, wobei zwei separate Teilfraktionen erhalten werden. Insbesondere die zweite Teilfraktion ist reich an Theanin, Theogallin und im Grüntee enthaltenen Aminosäuren.

In einer weiteren Stufe - Schritt e) - wird die betreffende Fraktion auf einen Trockensubstanz-Gehalt von ca. 50% konzentriert und kann bereits als solche verwendet werden. Der so enthaltene konzentrierte Theanin- und Theogallin-angereicherte Grüntee-Extrakt kann in einem weiteren Prozess-Schritt sprühgetrocknet werden.

Der wie oben beschrieben, hergestellte Grüntee-Extrakt weist im Wesentlichen folgende Zusammensetzung bezogen auf Trockensubstanz auf:

| | |
|---|---|
| Wassergehalt | 3,2% |
| Aschegehalt | 24,1 % |
| Summe Aminosäuren | 3,52% |
| Koffein | ≤ 0,01% |
| L-Theanin | 9,71% |
| Theogallin | 4,65% |

Der Rest besteht aus einer Vielzahl von Stoffen. Analysen haben als Restanteile Gerbstoffe zu 2,5 % sowie Kohlehydrate, Säuren und Säurederivate zu 52,3 % ergeben.

Verschiedene Tier- als auch eine Humanstudie haben überraschender Weise gezeigt, dass ein nach dem oben beschriebenen Verfahren hergestellter entkoffeinierter, Theanin und Theogallin angereicherter Grüntee-Extrakt die o.g. Eigenschaften aufweist, wobei insbesondere Theogallin, bzw. dessen Metaboliten, wie z.B. Chinasäure, für die Steigerung der cognitiven Leistungsfähigkeit verantwortlich ist. Theanin zeigt in dem beschriebenen Extrakt leicht relaxierende Eigenschaften.

Ferner konnte die Fähigkeit des entkoffeinierten, Theanin- und Theogallinangereicherten Grüntee-Extraktes zur Steigerung der Glukose-Aufnahmefähigkeit eines Zelltyps, nämlich aus murinen Fibroplasten differenzierte Adipocyten, in einem weiter unten beschriebenen Versuch nachgewiesen werden. Damit wird die Eignung des Grüntee-Extraktes als funktionales Lebensmittel zur Unterstützung des Glukose-Haushalts eines Menschen belegt.

Folgende experimentelle Studien wurden durchgeführt:
- Prüfung des Extraktes mittels eines Getränkes im Humanversuch
   Es wurde eine doppelblinde randomisierte, cross-over-placebokontrollierte, humanphysiologische Studie mit zwölf Probanden durchgeführt. Das Verumgetränk enthielt 2,57g/500 ml erfindungsgemäßen Grüntee-Extrakt, das Placebogetränk war frei von diesem Extrakt.
   Als Messparameter bzw. Hauptzielgrößen wurde die spektrale EEG-Leistung und das visuell evozierte Potential P300 untersucht. Als Methoden wurden das quantitativ-topographische EEG unter Anwendung der sogenannten "Cateem®"-Untersuchung sowie für die P300-Bestimmung das Auswertungsverfahrens "Caterpa" eingesetzt.
   Die Kriterien "Konzentrationszunahme", "Zunahme der Relaxation", "Steigerung der mentalen Leistungsfähigkeit" und "verbesserte cognitive Funktion" zeigen aufgrund der vorstehenden Auswertungen signifikante Verbesserungen bei Anwendung des Verumgetränkes im Vergleich zum Placebo.
   Insgesamt konnte damit eine Verbesserung der cognitiven Funktion bei gleichzeitiger Relaxation nach Aufnahme des Verumgetränkes gezeigt werden.
- Prüfung des Gesamt-Extrakts und einzelner Inhaltsstoffe am isolierten Rattenhirn-Hippocampus-Schnitt-Präparat:
   Als Präparate wurden Schnittpräparate analog Dimpfel et. al, 1991 verwendet, die einer elektrischen Stimulation unter Aufzeichnung der extrazellulären Feldpotentiale unterworfen wurden. Die gemessenen Feldpotentiale wurden nach einer Single-Stimuli-Anregung bzw. Theta-Burst-Stimulation aufgezeichnet.
   Es wurden folgende Substanzen untersucht:
   - entkoffeinierter, Theanin- und Theogallin- angereicherter Grüntee-Extrakt
   - Theogallin 97 %
   - Aminosäuremischung analog Grüntee-Extrakt
   - Aminosäuremischung analog Grüntee-Extrakt mit Theanin
   - L-Theanin >90 %
   - Chinasäure 98 %
   - Gallussäure 98 %

   Die in-vitro Hippocampus-Schnitt-Untersuchungsmethode wurde gewählt, um den Einfluss des im Humanversuch geprüften Grüntee-Extraktes und einzelner Inhaltsstoffe davon auf Hippocampus-Nervenzellgewebe zu untersuchen. Die Ergebnisse zeigen, dass der vorliegende Theanin- und Theogallin- angereicherte Grüntee-Extrakt in der Lage ist, das physiologische Muster der elektrischen Hippocampus-Aktivität zu verändern.
   Es konnte auch gezeigt werden, dass mehrere Substanzen des Grüntee-Extraktes die elektrische Aktivität beeinflussen. Glutaminsäure und Theogallin und dessen Metaboliten wie z.B. Chinasäure, sind als anregende Stoffe aufzufassen. Theanin zeigt gegenteilige Wirkung.
   Die Wirkung von Theogallin und dessen Metaboliten, wie z.B. Chinasäure als die cognitive Leistungsfähigkeit verbessernde Substanzen wurden bisher nicht beschrieben. Die Ergebnisse zeigen eine konzentrationsabhängige Stimulation am Hippocampus-Schnitt-Präparat, was zu einer Zunahme der Langzeitpotenzierung führt, einem Surrogatparameter für eine Aktivierung des räumlichen und zeitlichen Arbeitsgedächtnisses.
   Die Ergebisse ergänzen und unterstützen also die im Humanversuch erhaltenen Ergebnisse, wo ebenfalls eine Steigerung der cognitiven Leistungsfähigkeit bei gleichzeitiger Relaxation festgestellt werden konnte.
- Ergänzende in-vivo Experimente an freilaufenden Ratten mittels quantitativer Erfassung der elektrischen Himtätigkeit (Tele-Stereo-EEG).
   Beobachtet wurden adulte, freilaufende Fischer-344-Ratten mit implantierten 4-bipolaren konzentrischen Gehimelektroden. Es wurde eine kontinuierliche in-vivo-Analyse des elektrischen Feldpotentials (Tele-Stereo-EEG) des vorderen Cortex, Hippocampus, Striatum und der reticulären Formation analog Dimpfel et. al 1986 durchgeführt. Ferner wurde die Mobilität der Ratten mit einem Video Tracking System der GJB Datentechnik GmbH, Langewiesen gemessen.
   Als Substanzen wurden eingesetzt
   - Theanin- und Theogallin- angereicherter Grüntee-Extrakt
   - Glutaminsäure
   - L-Theanin > 90%
   - Theogallin 97%
   - Chinasäure 98%

   Das Modell "Tele-Stereo-EEG" an freilaufenden Ratten wurde als in-vivo Modell gewählt, um im Anschluss an die im Ratten-Hippocampus-in-vitro-Versuch erhaltene Ergebnisse zu überprüfen, insbesondere die Überwindung der Blut-Hirn-Schranke für die beschriebenen Substanzen.
   Die orale Gabe des Gesamt-Extraktes zeigt eine zeitlich abhängige, deutliche Stimulation der Feldpotenziale, gekennzeichnet durch Abnahme der Delta-, Theta- und Alpha-Frequenzen. Von den getesteten, im Extrakt vorliegenden Einzelsubstanzen sind Theanin, Theogallin bzw. Chinasäure die Substanzen, die im Wesentlichen zu den gemessenen Ergebnissen beitragen. Die im Ratten-Tele-Stereo-EEG erzielten in-vivo-Ergebnisse, deuten somit darauf hin, dass der beschriebene Grüntee-Extrakt bzw. die in dem Extrakt enthaltenen Komponenten auch beim Menschen die Blut-Hirn-Schranke überwinden können.
   Die Ergebnisse unterstützen damit die im Human-Versuch erhaltenen Daten, wo gezeigt wurde, dass der vorliegende Grüntee-Extrakt zu einer Steigerung der cognitiven Leistungsfähigkeit bei gleichzeitiger Relaxation führt.
- Glukose-Aufnahmetest als in-vitro-Untersuchung:
   Für einen Glukose-Aufnahmetest wurden 3T3-L1 Zellen (murine Fibroblasten) in drei Differenzierungsschritten während 8-9 Tagen mit Insulin (1µg/ml), Dexamethasone (40µM) und IBMX (500µM) zu Adipocyten differenziert. Beim zweiten Differenzierungsschritt wurden die Zellen (60000 Zellen/well) in Zellkulturplatten ausgesät. Für den Assay wurden die Zellen in Serum-freiem Medium für 3 Std. inkubiert. Nach Vorinkubation der Zellen mit einer 0.16 µM Insulinlösung und den Grüntee-Extrakten für 25 min bei 37°C wurde der Assay mit 2-Desoxy-D-[1-³H]-Glukose (0.11µCi/ml) gestartet und für 25 min bei Raumtemperatur inkubiert. Die Menge der von den Zellen aufgenommenen 2-Desoxy-D-[1-³H]-Glukose wurde nach diversen Waschschritten und Lysis der Zellen mittels eines Scintillationszählers bestimmt. Die Messungen wurden in einen Tri-Carb 1900 TR Liquid Scintillation Counter (Packard, USA) durchgeführt. Nullkontrollen (t(0)) wurden ohne Zugabe von Insulin durchgeführt. Als Negativkontrolle wurde Cytochalasin B (200µM), ein Inhibitor des Insulin-stimulierten Glukosetransporter Systems, verwendet. Das Ergebnis der Untersuchungen zeigt, dass der geprüfte Grüntee-Extrakt die Glukoseaufnahme in der beschriebenen Inkubationszeit im Vergleich zur Kontrolle mehr als verdoppelt. Dies trifft für alle drei eingesetzten Prüfkonzentrationen von 3 µg/ml, 100 ng/ml, sowie 300 pg/ml zu.

## Patentansprüche

1. Grüntee-Extrakt, insbesondere zur Verwendung als funktionelles Lebensmittel, Nahrungsergänzungsmittel oder entsprechende Zutat, mit einem angereicherten Anteil an Theanin, wobei der Extrakt ferner einen angereicherten Anteil an Theogallin enthält und entkoffeiniert ist, **gekennzeichnet durch** folgende Anteilsbereiche bezogen auf die Trockensubstanz:
- Theanin mindestens 6,0 %
- Theogallin mindestens 3,0 %
- Koffein maximal 0,03 %.

2. Grüntee-Extrakt nach Anspruch 1, **gekennzeichnet durch** folgende Anteile bezogen auf die Trockensubstanz:
- Wassergehalt 2,0 % bis 5,0 %
- Asche 20,0% bis 30,0 %
- Aminosäuren 2,0 % bis 10,0%
- Koffein unter 0,03 %
- Theanin 6,0% bis 40,0% und
- Theogallin 3,0 % bis 20,0%.

3. Grüntee-Extrakt nach Anspruch 2, **gekennzeichnet durch** folgende Zusammensetzung bezogen auf die Trockensubstanz:
- Wassergehalt 2,5 % bis 4,0 %, insbesondere 3,2%
- Asche 23,0 % bis 25,0 %, insbesondere 24,1 %
- Aminosäuren 3,0 % bis 4,0%, insbesondere 3,52 %
- Koffein unter 0,01 %
- Theanin 9,0 % bis 10,5 %, insbesondere 9,71 % und
- Theogallin 4,0 % bis 5,5 %, insbesondere 4,65 %.

4. Verwendung des Grüntee-Extraktes nach einem der vorgepannten Ansprüche als funktionelles Lebensmittel, Nahrungsergänzungsmittel oder Zutat zu einem solchen.

5. Verfahren zur Herstellung eines Grüntee-Extrakts nach einem der Ansprüche 1 bis 3, **gekennzeichnet durch** folgende Verfahrensschritte:
a) wässrige Extraktion von Grüntee-Blättern,
b) Flüssig-Flüssig-Extraktion des in Schritt a) erhaltenen wässrigen Grüntee-Extraktes mittels Ethylacetat unter Entfernung von Koffein und einem Teil der Polyphenole
c) absorptive Anreicherung des in Schritt b) gewonnenen, wässrigen, mit Theanin und Theogallin angereicherten Extraktes in einem mit funktionalisiertem Divinylbenzol befüllten Absorber,
d) Elution des Absorbers mit demineralisiertem Wasser, wobei zwei separate Teilfraktionen erhalten werden, und
e) Aufkonzentrieren des Eluats der zweiten, an Theanin, Theogallin und Grüntee-spezifischen Aminosäuren reichen Teilfraktion zu einem entkoffeinierten, mit Theanin und Theogallin angereicherten Grüntee-Extrakt mit einem Trockensubstanz-Gehalt von vorzugsweise mind. 50 %.

6. Verfahren nach Anspruch 5, **dadurch gekennzeichnet, dass** der in Schritt e) erhaltene Grüntee-Extrakt sprühgetrocknet wird.

## Claims

1. Green tea extract, in particular for use as a functional foodstuff, food supplement or corresponding ingredient, having an enriched content of theanine, wherein the extract additionally contains an enriched content of theogallin and is decaffeinated, **characterised by** the following content ranges relative to the dry matter:
- theanine at least 6.0%
- theogallin at least 3.0%
- caffeine maximum of 0.03%

2. Green tea extract according to claim 1, **characterised by** the following contents relative to the dry matter:
- water content 2.0% to 5.0%
- ash 20.0% to 30.0%
- amino acids 2.0% to 10.0%
- caffeine below 0.03%
- theanine 6.0% to 40.0%, and
- theogallin 3.0% to 20.0%.

3. Green tea extract according to claim 2, **characterised by** the following composition relative to the dry matter:
- water content 2.5% to 4.0%, in particular 3.2%
- ash 23.0% to 25.0%, in particular 24.1 %
- amino acids 3.0% to 4.0%, in particular 3.52%
- caffeine below 0.01 %
- theanine 9.0% to 10.5%, in particular 9.71% and
- theogallin 4.0% to 5.5%, in particular 4.65%.

4. Use of the green tea extract according to one of the preceding claims as a functional foodstuff, food supplement or ingredient of the latter.

5. Method for the production of a green tea extract according to one of claims 1 to 3, **characterised by** the following method steps:
a) aqueous extraction of green tea leaves,
b) liquid-liquid extraction of the aqueous green tea extract obtained in step a) by means of ethyl acetate with the removal of caffeine and a portion of the polyphenols,
c) absorptive enrichment of the aqueous extract obtained in step b) enriched with theanin and theogallin in an absorber filled with functionalised divinylbenzene,
d) elution of the absorber with demineralised water, wherein two separate part fractions are obtained and,
e) enrichment of the eluate of the second part fraction rich in theanine, theogallin and green tea specific amino acids to a decaffeinated, green tea extract enriched with theanin and theogallin with a dry matter content of preferably at least 50%.

6. Method according to claim 5, **characterised in that** the green tea extract obtained in step e) is spray-dried.

## Revendications

1. Extrait de thé vert, en particulier pour une utilisation en tant que produit alimentaire fonctionnel, complément alimentaire ou ingrédient correspondant, comprenant une part enrichie en théanine, l'extrait contenant en outre une part enrichie en théogalline et étant décaféinée, **caractérisé par** les domaines de proportions suivants par rapport à la substance sèche :
- théanine au moins 6,0 %
- théogalline au moins 3,0 %
- caféine au maximum 0,03 %

2. Extrait de thé vert selon la revendication 1 **caractérisé par** les proportions suivantes par rapport à la substance sèche :
- teneur en eau 2,0 % à 5,0 %
- cendres 20,0 % à 30,0 %
- acides aminés 2,0 % à 10,0 %
- caféine inférieure à 0,03 %
- théanine 6,0 % à 40,0 %, et
- théogalline 3,0 % à 20,0 %.

3. Extrait de thé vert selon la revendication 2 **caractérisé par** la composition suivante par rapport à la substance sèche :
- teneur en eau 2,5 % à 4,0 %, en particulier 3,2 %
- cendres 23,0 % à 25,0 %, en particulier, 24,1 %
- acides aminés 3,0 % à 4,0 %, en particulier, 3,52 %
- caféine inférieure à 0,01 %
- théanine 9,0 à 10,5 %, en particulier, 9,71 %, et
- théogalline 4,0 % à 5,5 %, en particulier, 4,65 %.

4. Utilisation de l'extrait de thé vert selon l'une des revendications précitées en tant que produit alimentaire fonctionnel, complément alimentaire ou ingrédient d'un tel produit.

5. Procédé de fabrication d'un extrait de thé vert selon l'une des revendications de 1 à 3, **caractérisé par** les étapes de procédé suivantes :
a) extraction aqueuse des feuilles de thé vert
b) extraction liquide-liquide de l'extrait aqueux obtenu dans l'étape a) au moyen d'acétate d'éthyle moyennant l'enlèvement de la caféine et d'une partie des polyphénols,
c) enrichissement par absorption de l'extrait aqueux, enrichi de théanine et de théogalline, obtenu dans l'étape b), avec un absorbant rempli de divinylbenzol fonctionnalisé,
d) élution de l'absorbant avec de l'eau déminéralisée, deux fractions partielles séparées étant obtenues, et
e) concentration de l'éluat de la deuxième fraction partielle riche en théanine, en théogalline et en acides aminés spécifiques au thé vert pour donner un extrait de thé vert décaféiné, enrichi en théanine et en théogalline comportant une teneur en substance sèche de préférence d'environ 50 %

6. Procédé selon la revendication 5 **caractérisé en ce que** l'extrait de thé vert obtenu dans l'étape c) est séché par pulvérisation.
